# EUROPÄISCHE PATENTSCHRIFT

(11) **EP 1 303 286 B1**
(45) Veröffentlichungstag und Bekanntmachung des Hinweises auf die Patenterteilung: **26.04.2006**
(21) Anmeldenummer: 01949489.7
(22) Anmeldetag: 11.07.2001
(51) Int. Cl.: A61K 35/76, A61P 31/12, A61P 35/00, A61P 37/00

(54) **VERWENDUNG VON STÄMMEN DES PARAPOXVIRUS OVIS ZUR HERSTELLUNG VON ANTIVIRALEN ARZNEIMITTELN UND ARZNEIMITTELN GEGEN KREBS**
USE OF STRAINS OF THE PARAPOX OVIS VIRUS FOR PRODUCING ANTIVIRAL PHARMACEUTICALS AND ANTICANCER PHARMACEUTICALS
UTILISATION DE SOUCHES DU VIRUS PARAPOX OVIS POUR LA FABRICATION DE MEDICAMENTS ANTIVIRAUX ET ANTICANCEREUX

(30) Priorität: 11.07.2000 DE 10033582; 09.05.2001 DE 10122451
(43) Veröffentlichungstag der Anmeldung: 23.04.2003
(73) Patentinhaber: Bayer HealthCare AG, 51368 Leverkusen (DE)
(72) Erfinder: WEBER, Olaf, Woodbridge, CT 06525 (US); SCHLAPP, Tobias, 51061 Köln (DE); SIEGLING, Angela, F-75015 Paris (FR); KNORR, Andreas, 40699 Erkrath (DE); HIRTH-DIETRICH, Claudia, 42115 Wuppertal (DE); THEISS, Gudrun, 42329 Wuppertal (DE); VOLK, Hans-Dieter, Prof.Dr., 10178 Berlin (DE)
(86) Internationale Anmeldenummer: PCT/EP2001/007991
(87) Internationale Veröffentlichungsnummer: WO 2002/004002

(56) Entgegenhaltungen:
- WO-A-95/22978
- WO-A-97/38724
- DE-A- 3 504 940
- RZIHA H-J ET AL: "Parapoxviruses: potential alternative vectors for directing the immune response in permissive and non-permissive hosts" JOURNAL OF BIOTECHNOLOGY, ELSEVIER SCIENCE PUBLISHERS, AMSTERDAM, NL, Bd. 73, Nr. 2-3, 20. August 1999 (1999-08-20), Seiten 235-242, XP004180185 ISSN: 0168-1656

## Beschreibung

Die vorliegende Erfindung betrifft die Verwendung eines Stammes des Parapoxvirus ovis für die Herstellung von Arzneimitteln zur Anwendung bei Mensch und Tier als Immuntherapeutikum bei Immunschwächen infektiöser oder nicht infektiöser Natur, sowie zur Behandlung von Tumorerkrankungen, viralen Infektionen und damit einhergehenden Erkrankungen.

Die vorliegende Erfindung betrifft weiter die Verwendung eines Stammes des Parapoxvirus ovis zur Herstellung von Arzneiformen zur Verwendung als Immuntherapeutika oder Immunprophylaktika, bei der Stressmetaphylaxe zur Verhinderung oder Vorbeugung von Infektionskrankheiten nach Stress (z.B. Operationen); zur Verwendung bei der Infektionsprophylaxe zur Verhinderung oder Vorbeugung von Infektionskrankheiten durch die Gabe vor Operationen oder Eingriffen (z.B. vor Implantation von Prothesen oder vor zahnärztlichen Eingriffen), zur Verwendung bei der Infektionsmetaphylaxe oder Therapie akuter oder chronischer viraler Infektionen beispielsweise des Respirationstraktes, der Papillomvirusinfektionen, der Infektion mit Herpesviren, der HIV - Infektion, der Virusinfektion innerer Organe wie beispielsweise der Infektion mit Hepatitisviren, wobei im Rahmen der Erfindung als Stamm von Parapoxvirus ovis der Stamm NZ2 verwendet wird.

Verwendet werden können weiter durch Passagierung und/oder Adaptation an bestimmte Zellen, beispielsweise WI-38, MRC-5 oder Vero-Zellen erhaltene Abkömmlinge dieses Stammes bzw. Teile oder Bruchstücke von Viren dieses Stammes oder dessen Abkömmlingen. Unter Teilen sind mittels geeigneter Vektoren, beispielsweise Vaccinia, in geeigneten Systemen beispielsweise Fibroblastenzellkulturen exprimierte genomische oder subgenomische Fragmente zu verstehen. Unter Bruchstücken versteht man die durch biochemische Aufreinigung, beispielsweise durch Chromatografie, erhaltenen Fraktionen physikalisch, beispielsweise durch Einwirkung von Ultraschall, aufgeschlossener Partikel.

Die vorliegende Erfindung betrifft weiter die Verwendung des besagten Stammes von Parapoxvirus ovis zur Herstellung von Arzneimitteln und pharmazeutischen Zubereitungen. Darüber hinaus betrifft die Erfindung die Verwendung des besagten Stammes von Parapoxvirus ovis in Kombination mit anderen Mitteln zur Herstellung von Arzneimitteln und pharmazeutischen Zubereitungen zur antiviralen- oder Krebstherapie.

Es ist bekannt, dass latente und chronisch persistente virale Infektionen durch eine Immunsuppression aktiviert bzw. reaktiviert werden können, oder umgekehrt, dass das Immunsystem die akute Erkrankung, die durch ein Virus, das latent ist, hervorgerufen werden kann, unterdrückt (z.B. rekurriert eine latente Herpesvirus-Infektion bei Immunsuppression: Lippenbläschen bei Stress oder Kortisongabe). Es ist weiter bekannt, dass chronisch persistente und latente virale Infektionen schwer oder gar nicht mit herkömmlichen antiviralen Substanzen auf niedermolekularer Basis therapierbar sind.

Ein Grund dafür kann die fehlende virale enzymatische Aktivität bei solchen Infektionen sein (beispielsweise das Fehlen einer viralen Polymerase-Aktivität, die einen nukleosidischen Inhibitor erst in die virale Nukleinsäure einbauen muss, damit dieser z.B. zum Kettenabbruch in der viralen DNA führen kann; beispielsweise das Fehlen einer viralen Thymidinkinase-Aktivität, die z.B. eine antivirale Verbindung erst phosphorylieren muss, damit diese aktiv werden kann) oder aber die fehlende Erkennung infizierter oder entarteter Zellen, z.B. Krebszellen oder viraler Antigene durch das Immunsystem des Wirtes.

Bekannt ist ebenfalls, dass bei chronisch persistierenden viralen Infektionen eine Superinfektion mit einem anderen Virus zu antiviralen, gegen das chronisch persistierende Virus gerichteten Effekten führen kann.¹⁾ Die Abhängigkeit dieses Effektes von Interferonen wie IFN-,γ und TNF-α, die von T-Zellen, natürlichen Killerzellen und Makrophagen sezerniert werden, konnte von den Autoren ¹⁾ gezeigt werden.

Die Ergebnisse dieser Autoren bestätigte eine andere frühere Studie, in der gezeigt wurde, dass Class-I-restringierte cytotoxische T-Zellen die hepatozelluläre HBV-Genexpression in HBV- transgenen Mäusen hemmen konnten, dass dieser Prozess ohne Zerstörung der Leberzellen ablief und dass der Prozess durch TNF-α und IFN-γ hervorgerufen wurde ²⁾.

In der tiermedizinischen Praxis wird seit längerer Zeit ein Produkt zur Induktion "paraspezifischer Immunität" ein sogenannter Paramunitätsinducer therapeutisch, meta- und prophylaktisch eingesetzt. Paramunitätsinducer bestehen z.B. aus chemisch inaktiviertem Parapoxvirus ovis, Strain D 1701 (DE 3 504 940). Ein auf der Basis dieses Virus (Parapoxvirus ovis, Strain D 1701) hergestelltes Produkt ist BAYPAMUN®.

Das inaktivierte Parapoxvirus induziert im Tier einen unspezifischen Schutz gegenüber Infektionen mit den verschiedensten Erregern. Man nimmt an, dass dieser Schutz über verschiedene Mechanismen des organismus-eigenen Abwehrsystems vermittelt wird.

Zu diesen Mechanismen zählen die Induktion von Interferon, die Aktivierung der natürlichen Killerzellen, die Induktion der "Kolonien-Stimulierenden Aktivität" (CSA), sowie die Stimulierung der Lymphozytenproliferation. Frühere Untersuchungen zum Wirkmechanismus zeigten die Stimulation von Interleukin 2 und Interferon-α ³⁾.

Vor diesem Hintergrund stellt sich daher die Aufgabe, die therapeutische Nutzbarkeit der ausgezeichneten Wirkung von Parapoxvirus ovis dahingehend weiter zu verbessern, dass die oben beschriebene generalisierte sogenannte Induktion einer paraspezifischen Immunantwort des Parapoxvirus ovis, Stamm D 1701 qualitativ erhöht und dahingehend verbessert wird, dass durch Einsatz geringerer Dosen bessere antivirale oder anti-Tumorwirkungen erzielt werden können. Dies ließe zusätzlich einen nebenwirkungsärmeren therapeutischen Effekt erwarten.

Aufgabe der Erfindung war es deshalb, die immunologische Wirkung des Parapoxvirus zu verbessern. Die Aufgabe wird gelöst durch Verwendung der oben genannten Stämme von Parapoxvirus ovis anstelle des herkömmlich verwendeten Strain D 1701.

Die vorliegende Erfindung betrifft die Verwendung von Viren, die taxonomisch zu dem Stamm NZ2 von Parapoxvirus ovis gehören, zur Herstellung von Arzneimitteln gegen virale Infektionen und Krebs bei Mensch und Tier.

Weiterhin betrifft die Erfindung die Verwendung von durch Passagierung oder Adaptation an geeigneten Zellsystemen wie beispielsweise humanen Zellen wie WI-38, MRC-5, Affenzellen, z.B. Vero-Zellen, bovine Zellen wie z.B. BK-K13A47/Reg oder MDBK, und ovinen Zellen wie MDOK, erhaltenen Abkömmlingen des erfindungsgemäßen Stammes zur Herstellung von Arzneimitteln gegen virale Infektionen und Krebs bei Mensch und Tier zum Gegenstand hat, und weiter die Verwendung von Teilen oder Bruchstücken des genannten Stammes und seiner Passagierungs- und Adaptationsvarianten, wobei unter Teilen mittels geeigneter Vektoren wie Vacciniaviren in geeigneten Systemen wie Fibroblastenzellkulturen exprimierte genomische oder subgenomische Fragmente und unter Bruchstücken die durch biochemische Aufreinigung wie Chromatografie erhaltenen Fraktionen der exprimierten oder physikalisch aufgeschlossenen viralen Partikel zu verstehen sind, zur Herstellung von Arzneimitteln gegen virale Infektionen und Krebs bei Mensch und Tier zum Gegenstand hat, und weiter die Verwendung des Stammes von Parapoxvirus ovis und wie vorstehend beschrieben abgeleiteten Derivate zur Herstellung von Arzneimitteln und pharmazeutischen Zubereitungen als Immuntherapeutika oder Immunprophylaktika bei akuten und chronischen viralen Infektionen des Respirationstraktes und der inneren Organe, zum Gegenstand hat, und weiter die Verwendung des Stammes und abgeleiteten Derivate zur Herstellung von Arzneimitteln und pharmazeutischen Zubereitungen für die Stressmetaphylaxe und zur Verhinderung oder Vorbeugung von Infektionskrankheiten nach Stress sowie bei der Infektionsprophylaxe im Rahmen von Operationen und zahnärztlichen Eingriffen zum Gegenstand hat, und weiter die Verwendung des Stammes und abgeleiteten Derivate zur Herstellung von Arzneimitteln und pharmazeutischen Zubereitungen für die Verwendung bei der Infektionsmetaphylaxe oder Therapie akuter und chronischer viraler Infektionen beispielsweise des Respirationstraktes, der Papillomvirusinfektionen, der Infektion mit Viren der Herpesgruppe, der HIV-Infektion, der Virusinfektion innerer Organe wie beispielsweise der Infektion mit Hepatitisviren und weiter die Verwendung des Stammes und abgeleiteter Derivate zur Herstellung von Arzneimitteln und pharmazeutischen Zubereitungen für die Verwendung gegen Krebs zum Gegenstand hat, und weiter die Verwendung des Stammes und davon abgeleiteter Derivate in Kombination mit anderen Mitteln zur Herstellung von Arzneimitteln und pharmazeutischen Zubereitungen zur antiviralen- oder Krebstherapie bei Mensch und Tier zum Gegenstand hat.

Ein bevorzugter Gegenstand der Erfindung ist die Verwendung des Stammes von Parapoxvirus ovis in Kombination mit anderen Mitteln zur Herstellung von Arzneimitteln und pharmazeutischen Zubereitungen für die orale Applikation, und/oder in einer magensaftresistenten Formulierung für die orale Applikation.

Der hier genannte Parapoxvirus ovis NZ-2 wurde am 10. Juli 2001 bei der European Collection of Cell Cultures, Centre for Applied Microbiology and Research, Porton Down, Salisbury, Wiltshire, SP4 0JG,United Kingdom hinterlegt. Die Hintelegungsnummer lautet ECACC-01071006.

### Beispielhaft wurde gefunden:

### 1. Nachweis der therapeutischen Wirksamkeit in Hepatitis B Virus - transgenen Mäusen

Für den "proof of concept" Versuch wurden Hepatitis B Virus transgene Mäuse (Stamm HBV 1.3 X⁻tg,) verwendet. Es wurden 7 männliche Tiere im Alter von 8 bis 10 Wochen je Gruppe verwendet. Die Applikation der verschiedenen Dosen erfolgte intraperitoneal in einem Volumen von 0.15 ml am Tag 1 (Beginn des Experimentes) und Tag 4.

Für die Applikation wurden folgende Verdünnungen eines jeden Virus-Strains für die einzelnen Dosisgruppen hergestellt:

| | |
|---|---|
| 1. Dosis | 1,5x10⁶ TCID₅₀ |
| 2. Dosis | 5x10⁵ TCID₅₀ |
| 3. Dosis | 1,5x10⁵ TCID₅₀ |
| 4. Dosis | 5x10⁴ TCID₅₀ |

Als Placebo-Kontrolle diente steriles, pyrogenfreies PBS.

Die Aufkonzentration der Viren wurde folgendermaßen vorgenommen: 500 ml Überstand aus Parapoxvirus ovis, strain NZ 2 (Titer ca. 2x10⁵ TCID₅₀/ml) und Parapoxvirus ovis, strain D 1701 (Titer 1x10⁷ TCID₅₀/ml) wurden auf denselben Virustiter eingestellt. Dafür wurde eine Beckmann Ultrazentrifuge (Rotor SW28 bei 28000 RPM 3 Std. 4°C) benutzt. Nach der Zentrifugation wurden die Pellets mit entsprechenden Volumina Verdünnungsmedium auf einen Titer von 1x10⁷ TCID₅₀/ml eingestellt.

Aliquots der entsprechenden Dosierungen wurden für eine kontrollierende Rücktitration verwendet, die die Titer dieser Dosierungen bestätigte. Die entsprechenden Dosen wurden nach Entnahme der Kontrollaliquots für 1 Stunde bei 56°C inaktiviert.

### Verdünnungsmedium:

10 ml EMEM 10x
2,7 ml Bicarbonat 2 g/l
1 ml Glutamin 1 %
86,3 ml Aqua-bidest

Am Tag 5 wurden die Tiere schmerzlos getötet und Leber und Blut entnommen. Etwa 20 mg Leber je Tier wurden mit QIAamp Tissue Kit (Qiagen, Hilden) aufgearbeitet, die Konzentration der DNA wurde photometrisch bestimmt und die Integrität in einem 1%igen Agarosegel elektrophoretisch kontrolliert. Die DNA wurde in einem Dot-Blot-Hybridisierungsverfahren mit einer HBV-spezifischen Sonde hybridisiert. Zuvor wurde die DNA mit RNAse A (Qiagen, Hilden) behandelt, um durch RNA hervorgerufene Signale auszuschließen. 200 µl DNA (10 µg) wurde auf eine Nylon-Membran (Boehringer Mannheim) gebracht, 4 mal für jeweils 3 Minuten mit Soak I (O,5N NaOH; 1M NaCI) und zwei mal mit Soak n (3M NaCi; 0,5M Tris-HCl, pH 7,4) behandelt, bei 120°C für eine halbe Stunde gebacken und im Anschluss mit einem Standard Hybridisierungspuffer (5xSSC, N-lauroylsarcosine, 0,1 % w/v; SDS, 0,02 %; Blocking reagent, 1x and 100 µg/ml fish sperm DNA) ohne Sonde für 30 Minuten bei 60°C prähybridisiert. Im Anschluss an diesen Schritt erfolgte die Hybridisierung mit einer random-Oligonukleotid geprimten Sonde, die das gesamte HBV Genom enthält (20-40 ng/ml Hybridisierungspuffer). Der Filter wurde danach für 10 Minuten bei 64°C in 4xSSC/0,1 %SDS, 2xSSC/0,1 %SDS und1xSSC/0,1 %SDS gewaschen.

Die immunologische Detektion erfolgte mit dem CDP-Star® - System (Boehringer Mannheim) entsprechend den Vorschriften des Herstellers. Für die Auswertung wurde ein LumiImager® (Boehringer Mannheim) verwendet. Die Quantifizierung HBV-spezifischer DNA im Blut erfolgte mittels quantitativer PCR. Zunächst wurde mittels Zentrifugation des EDTA-Blutes das Plasma gewonnen. Die DNA wurde mittels des HighPure 16 System Viral Nucleic Acid Kits (Boehringer Mannheim) aufgereinigt und durch quantitative PCR mit dem ABI PRISM™ 7700 Sequence Detection System (PE Applied Biosystems) auf HBV-spezifische Signale untersucht. Folgende Primer und Sonde wurden eingesetzt: Sonde:

Die DNA wurde in 50µl Reaktionsvolumen amplifiziert (die Reaktion enthielt 1,4 mM von jedem dNTP, 4.75 mM MgCl₂, 15 pmol eines jeden Primers und der Sonde, 5 µl 10-fach PCR Puffer [alle PCR Reagenzien stammten aus dem TaqMan core reagent kit; Perkin Elmer/Roche Molecular Systems Inc.] und 1.25 U Taq DNA polymerase und 0.25 U Amp Erase. Nach einem initialen Denaturierungsschritt (10 min bei 95°C), wurden die Proben 40 Zyklen Denaturierung (95°C 30 sec) und Annealing/Extension (56°C, 1 min) ausgesetzt. Die Analyse der Produkte erfolgte mit der ABI PRISM™ 7700 Sequence Detection System standard software.

Eine histochemische Analyse erfolgte mittels Antikörpern gegen das Hepatitis B Virus core Antigen (Dako). Dafür wurden Teile eines Leberlappens über Nacht in 4 % Formaldehyd fixiert, in Paraffin eingebettet und geschnitten (5µm). Nach Entparaffinisierung und Rehydration wurde die endogene Peroxydaseab-tivität mit 3 % H₂O₂ für einen Zeitraum von 20 Minuten gequencht. Unspezifische Bindung wurde mit Normal-Schafserum geblockt. Im Anschluss wurden die Schnitte 30 Minuten bei Raumtemperatur mit dem 1:500 verdünnten Antikörper inkubiert. Alle nachfolgen-. den Schritte wurden mit dem Vectastain ABC Kit (Vector Laboratories) wie vom Hersteller beschrieben durchgeführt.

Die Immunreaktion wurde mit 3.3 Diamino-benzidintetrachlorid und Wasserstoffperoxid visualisiert. Die Schnitte wurden Hematoxylin/Eosin gegengefärbt.

Die statistischen Analysen der Ergebnisse erfolgten durch Varianzanalyse und post hoc - Vergleich.

Im Ergebnis wurde überraschend festgestellt, dass man durch Verwendung des Strains NZ 2 eine Verstärkung der antiviralen Wirkung im Vergleich zu bekanntem Parapox ovis Strain D 1701 erreicht. Damit wird es erstmals möglich, mit Hilfe von Parapoxvirus ovis die komplexe Kapazität des Immunsystems in einer Stärke zu induzieren, die sich signifikant von der Wirkstärke der bisher bekannten Paramunitätsinducer unterscheidet.

Folgende Ergebnisse wurden überraschenderweise erhalten:

Leber: Bei mit NZ-2 behandelten Tieren wurde im Vergleich zu mit D 1701 behandelten Tieren eine signifikant stärkere Reduktion von HBV-spezifischer DNA festgestellt. Im Vergleich mit D 1701 ist NZ2 in seiner antiviralen Wirksamkeit potenter: In der höchsten Dosisgruppe reduziert die Gabe von NZ 2 die HBV-spezifische DNA um mehr als den Faktor 45 besser als die Gabe von gleicher Menge D 1701, in der niedrigsten Dosisgruppe um den Faktor 57. Im Plasma reduziert die Gabe von NZ 2 die HBV-spezifische DNA in der niedrigsten Dosisgruppe um mehr als den Faktor 10 besser als die Gabe von gleicher Menge D 1701. Damit wurde gezeigt, dass die therapeutische Wirksamkeit von Parapoxvirus ovis Strain NZ 2 signifikant besser als diejenige von Strain D 1701 ist.

In den Abbildungen wird die Reduktion HBV-spezifischer Signale im Vergleich zur Placebogruppe (diese wurde mit 100 % gleichgesetzt) aufgezeigt.

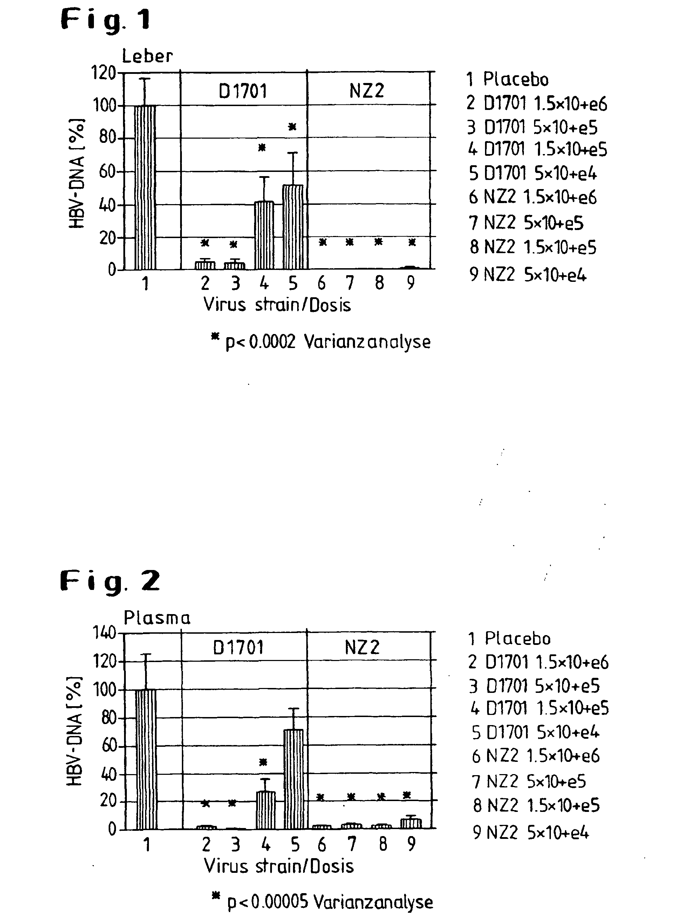

Abbildung 1 zeigt Ergebnisse der Behandlung HBV-transgener Mäuse mit Strain D 1701 oder NZ 2. HBV-spezifische DNA wird in der Leber in allen Dosisgruppen bei beiden Strains signifikant im Vergleich zur Placebogruppe reduziert, durch Strain NZ 2 aber stärker. Bei den beiden niedrigsten NZ 2-Dosierungen liegt eine signifikant stärkere Reduktion der HBV-spezifischen DNA als bei den äquivalenten D 1701 Dosisgruppen vor. In Abbildung 2 wird das Ergebnis der Behandlung HBV-transgener Mäuse mit Strain D 1701 oder NZ 2 im Plasma gezeigt. HBV-spezifische DNA wird im Plasma in allen Dosisgruppen bei beiden Strains signifikant im Vergleich zur Placebogruppe reduziert, durch Strain NZ 2 aber stärker. Im Gegensatz zur niedrigsten NZ 2 - Dosierung wirkt die geringste Dosis von Strain D 1701 nicht mehr signifikant antiviral.

### 2. Induktion von Zytokinen:

7 bis 8 Wochen alte weibliche Balb/c Mäuse wurden unter sterilen Bedingungen gehalten und für den Versuch verwendet. Die Tiere wurden randomisiert und in Gruppen mit jeweils 6 Tieren aufgeteilt. Folgendes Behandlungsschema wurde angewendet:
- Gruppe 1:: Placebo
- Gruppe 2:: Parapoxvirus ovis strain D 1701; 5x10⁴ TCID₅₀/Dosis
- Gruppe 3:: Parapoxvirus ovis strain NZ 2; 5x10⁴ TCID₅₀/Dosis
- Gruppe 4:: Placebo
- Gruppe 5:: Parapoxvirus ovis strain D 1701; 5x10⁴ TCID₅₀/Dosis
- Gruppe 6:: Parapoxvirus ovis strain NZ 2; 5x10⁴ TCID₅₀/Dosis

Das Applikationsvolumen betrug 10 ml/kg, die Applikation erfolgte intraperitoneal.

6 Stunden nach der Applikation wurden die Tiere der Gruppen 1 bis 3, 12 Stunden nach der Applikation die Tiere der Gruppen 4 bis 6 getötet, Peritonealzellen über eine Lavage mit eiskaltem PBS gewonnen sowie die portalen und mesenterialen Lymphknoten isoliert.

Die Peritonealzellen wurden über einen Zentrifugationsschritt (5 min bei 3000 rpm bei Raumtemperatur in einer Eppendorf-Tischzentrifuge) aufkonzentriert, anschließend in 0,2 ml Lysis-Puffer (Lysis-Lösung: 25 mM Natriumcitrat, 4 M Guanidiniumisothiocyanat, 0,5 % N-Lauroyl-Sarkosin) aufgenommen, schockgefroren und bis zur RNA-Präparation bei -75°C gelagert.

Die Präparation der totalen RNA erfolgte mittels saurer Phenol/Chloroform-Extraktion. Dazu wurden die in Lysispuffer eingefrorenen Proben bei Raumtemperatur aufgetaut und zur Extraktion mit folgenden Lösungen versetzt: 1/10 Lysispuffer-Volumen 2 m Natriumacetat (pH 4,0), 1 Lysispuffer-Volumen wassergesättigtes Phenol, 1/5 Lysisppuffer-Volumen Chloroform / Isoamylalkohol (24:1). Dieser Ansatz wurde für 10 Sekunden auf dem Vortexer vermischt und anschließend 10 Minuten auf Eis temperiert. Die Phasentrennung erfolgt durch Zentrifugation bei 15365 g und 4°C für 30 Minuten. Danach wurde die wässrige Phase in ein neues Gefäß überführt, und zur Isolation der in dieser Phase befindlichen RNA 8 ml RNA-MATRIX aus dem Rnaid™ plus Kit (DIANOVA) zugegeben und 15 Minuten bei Raumtemperatur inkubiert. Der entstandene RNA/RNA-MATRIX-Komplex wurde durch Zentrifugation bei 7000 g pelletiert und der Überstand verworfen. Das Pellet wurde nachfolgend 2x mit je 250 ml RNA-WASH (DIANOVA) gewaschen und nach dem letzten Waschen durch Vakuumzentrifugation getrocknet. Die RNA wurde durch Zugabe von 20-30 ml RNAse freiem destillierten Wasser durch Temperieren bei 55°C für 15 Minuten eluiert. Nach Zentrifugation bei 7000 g und Raumtemperatur für 1 Minute wurde die Matrix durch Überführen der RNA-Lösung in ein neues Gefäß abgetrennt.

Die Qualität der RNA wurde mittels Gelelektrophorese überprüft. Die RNA wurde bei -70°C gelagert.

Die Synthese der cDNA erfolgte durch reverse Transkription der mRNA unter Verwendung von Oligo(dT)-Primern als Startermoleküle für die Polymerisation. Im Synthese-Ansatz waren folgende Komponenten vorhanden: 200 ng-2 µg totale RNA, 2 µl reverse Transkriptase M-MLV (200 U/µl) (GIBCOBRL), 8 µl des zugehörigen 5xRT-Puffer (GIBCO/BRL), 1 µl DTT (0.1M) (GIBCOBRL), 4 µl dNTP (2,5 mM) (SIGMA), 2 µl Oligo(dT)12-18-Primer (100 µg/ml) (PROMEGA), 1 µl human plazental RNAse Inhibitor (10000 U/ml) (GIBCOBRL) und Wasser ad 40 µl Gesamtvolumen. Der Ansatz wurde 10 Minuten bei Raumtemperatur temperiert und anschließend 45 Minuten bei 37°C inkubiert, nachfolgend für 3 Minuten auf 95°C erhitzt und sofort auf Eis abgekühlt. Die auf diese Weise synthetisierte cDNA wurde bei -20°C gelagert.

Die Mengen der cDNAs wurden mittels eines "housekeeping" Gens (β-Aktin) standardisiert. Die quantitative PCR wurde mit dem ABI PRISM™ 7700 Sequence Detection System (PE Applied Biosystems) durchgeführt. Folgende Primer wurden eingesetzt:

Die DNA wurde in 25µl Reaktionsvolumen amplifiziert (die Reaktion enthielt 1,4 mM von jedem dNTP, 4 mM MgCl₂, 0,3 µmol eines jeden Primers und der Sonde, 2,5 µl 10-fach PCR Puffer mit SYBR Green [alle PCR Reagenzien stammten aus dem SYBR Green PCR core reagent kit; Perkin Elmer/Roche Molecular Systems Inc.] und 1.25 U Taq DNA polymerase und 0.25 U Amp Erase. Nach einem initialen Denaturierungsschritt (95°C für 10 min), wurden die Proben 40 Zyklen Denaturierung (95°C 30 s) und Annealing/Extension (60°C, 1,30 min) ausgesetzt. Die Analyse der Produkte erfolgte mit der ABI PRISM™ 7700 Sequence Detection System Standard Software. Die statistischen Analysen der Ergebnisse erfolgte mit der Varianzanalyse und post hoc - Vergleich.

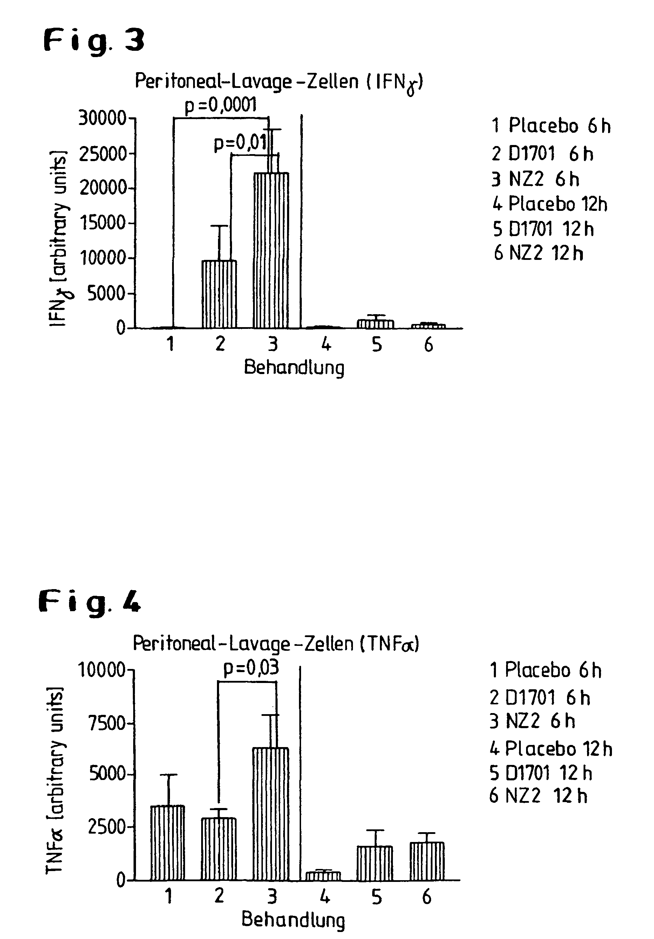

Folgende Ergebnisse wurden überraschenderweise erhalten:
1. Die Expression von Interferon γ wird nach Behandlung mit Strain D 1701 oder Strain NZ 2 6 und 12 Stunden nach der Applikation induziert (Abbildung 3). Diese Induktion ist bei Strain NZ 2 sowohl signifikant höher gegenüber Placebo, als auch gegenüber Strain D 1701. Die nach Gabe von D 1701 beobachtete Höhe der Interferon γ Expression ist nicht signifikant gegenüber der Placebokontrolle. Dargestellt sind die Werte, die in durch peritoneale Lavage erhaltenen Zellen gemessen wurden.
2. Die Expression von TNF α wird nach Behandlung mit Strain D 1701 12 Stunden oder nach Behandlung mit Strain NZ 2 6 und 12 Stunden nach der Applikation induziert, (wobei nach 12 Stunden bereits ein Absinken im Vergleich zum 6 Stunden-Wert festgestellt werden kann; Abbildung 4). Diese Induktion ist 6 Stunden nach der Applikation bei Strain NZ 2 signifikant höher als vergleichsweise gegenüber Strain D 1701. Dargestellt sind die Werte, die in durch peritoneale Lavage erhaltenen Zellen gemessen wurden.
3. Die Expression von IL-15 wird nach Behandlung mit Strain D 1701 oder mit Strain NZ 2 6 und 12 Stunden nach der Applikation induziert (Abbidung 5). Diese Induktion ist 6 Stunden nach der Applikation bei Strain NZ 2 signifikant höher als vergleichsweise gegenüber Strain D 1701 oder Placebo. Die nach Gabe von D 1701 beobachtete Höhe der IL-15 - Expression ist nicht signifikant gegenüber der Placebokontrolle. Dargestellt sind die Werte, die in durch peritoneale Lavage erhaltenen Zellen gemessen wurden.

### 3. Nachweis der therapeutischen Wirksamkeit in tumortragenden Nacktmäusen

MDA-MB231 Zellen (ATCC#HTB26) wurden in Komplettmedium (DMEM 88 5, FBS 10 %, Penicillin/Streptomycin 1%, L-Glutamin 1 % (jeweils Gibco Life Technologies)) bei 37°C und 5 % CO₂ in einem Inkubator kultiviert. Am Tage der Transplantation waren die Zellen zu ca. 70 % konfluent. Die Zellen wurden trypsiniert, mit HBSS gewaschen, gezählt und auf 2,5x10⁷ Zellen/ml mit vorgekühltem PBS eingestellt. Es wurden weibliche NCr nude Mäuse (taconic) verwendet. Die Mäuse waren zwischen 8 und 10 Wochen alt und wogen ca. 22 g. Alle Manipulationen an den Tieren wurden unter sterilen Bedingungen durchgeführt. Sx10⁶ Zellen wurden in einem Gesamtvolumen von 0,2 ml subcutan in die Flankenregion injiziert. Danach wurden die Mäuse weitere sieben Tage gehalten bis die Tumoren eine durchschnittliche Masse von ca. 80 mg erreicht hatten. Die Tumoren wurden vermessen und die Mäuse nach dem Zufallsprinzip in drei Gruppen zu jeweils zehn Tieren eingeteilt. Die einzelnen Gruppen erhielten wie folgt verabreicht:
- Gruppe 1:: Placebo (PBS)
- Gruppe 2:: Parapoxvirus ovis, Stamm D1701
- Gruppe 3:: Parapoxvirus ovis, Stamm NZ2

D1701 wurde in einer Dosis von 2,5x10⁵ TCID₅₀, NZ2 in einer Dosis von 1x10⁵ TCID₅₀ insgesamt jeweils vier mal im Abstand von jeweils drei Tagen verabreicht. Die Tumoren wurden zwei mal pro Woche vermessen. Signifikanzen wurden mittels Student's Test ermittelt.

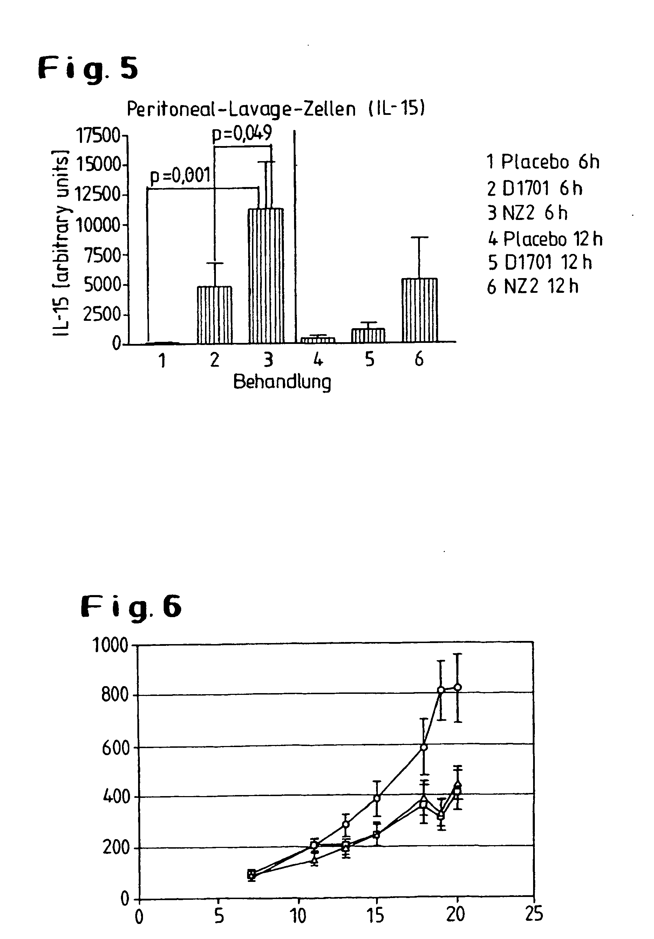

Figur 6 zeigt die mittlere Größe der Tumoren (in Milligramm) in Tieren der Gruppen 1 bis 3 über die Versuchszeit (in Tagen).
(Symbole: Gruppe 1, Kreise; Gruppe 2, Dreiecke; Gruppe 3, Quadrate)

Überraschender Weise wurde im experimentellen System eine im Vergleich zur Kontrollgruppe signifikante gegen Tumoren gerichtete Wirksamkeit gefunden (p<0,05). Dabei zeigte sich der Stamm NZ2 deutlich potenter als der Stamm D1701. Zum Erreichen des gleichen Effektes war nur ungefähr die halbe Menge NZ2, verglichen mit D1701, nötig.

Dieser Befund belegt deutlich die therapeutische Wirksamkeit von erfindungsgemäßen Viruspräparationen in tumortragenden Nacktmäusen.

Nacktmäuse sind immundefizient und verfügen nicht über funktionelle T-Zellen. Die gegen die Tumoren gerichtete Aktivität wird vermutlich im experimentellen System durch die natürlichen Klientelen (NK), andere Zellen und direkte Wirkung von Zytokinen/Chemokinen bedingt. Bei einem vollständigen und intakten Immunsystem wäre zu vermuten, dass die bessere Wirksamkeit von NZ2 noch deutlicher ersichtlich wird.

### 4. Weitere biologische Unterschiede zwischen NZ2 und D1701

Es wurde gefunden, dass Parapox Virus NZ2 im Gegensatz zum Stamm D1701 auf humanen Zelllinien fortgesetzt passagierbar ist. Dies zeigt grundsätzliche Unterschiede im Replikationsverhalten und/oder in den viralen Rezeptoren bei NZ2 und D1701.

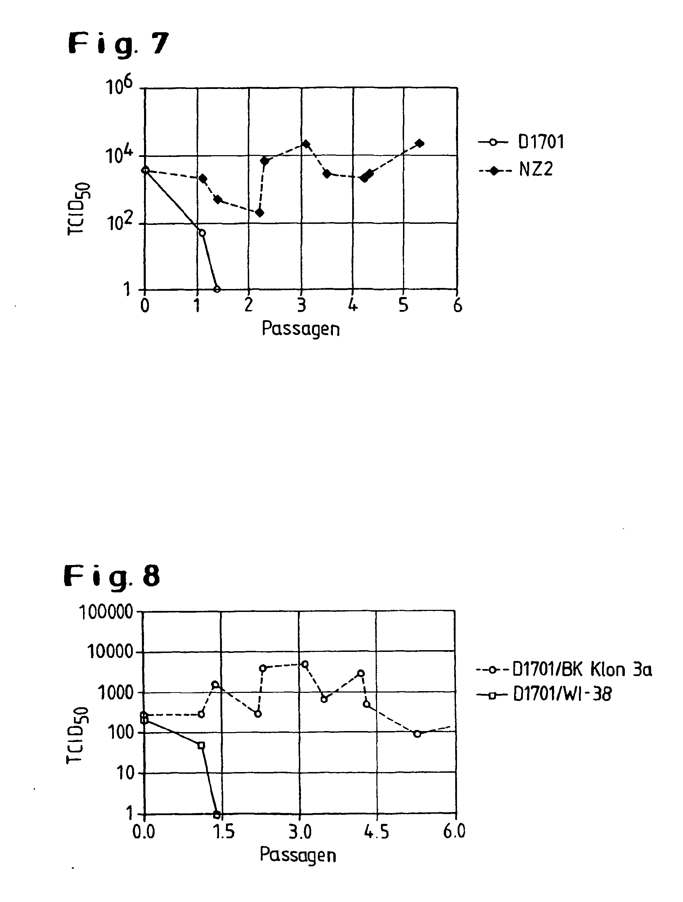

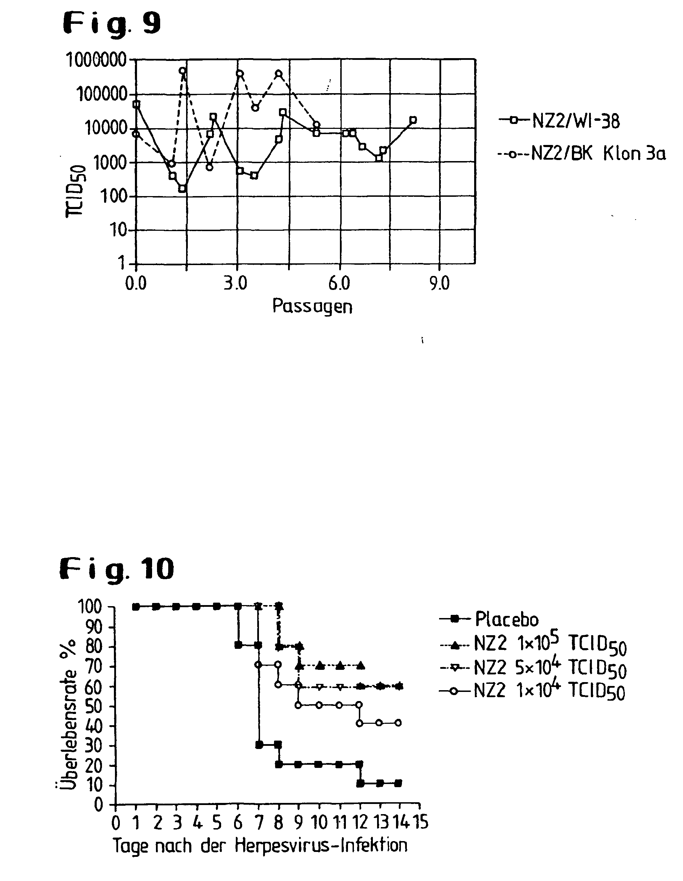

Die Adaptation an humane Zelllinien ist eine wichtige Voraussetzung für die Produktion von viralen Stämmen auf humanen Zelllinien.
4 .a) Fortgesetzte Passagierbarkeit auf humanen MRC-5 Zellen.
   Ein Adaptionsversuch der Stämme NZ2 und D1701 an die humane diploide Zelllinie MRC-5 ist in Figur 7 dargestellt. MRC-5 ist für die Produktion von biologischen Wirkstoffen und Impfstoffen geeignet. Die Stämme wurden mit dem gleichen Ausgangstiter eingesetzt und die entsprechenden Zellkulturüberstände über 5 Passagen in MRC-5 Zellen weitergefüha. Dargestellt ist die Austitration von Proben aus den Überständen der jeweils infizierten MRC5-Zellen in TCID₅₀ über die Passagennummer. Lediglich der Stamm NZ2, nicht aber D1701 war auf bovinen Nierenzellkulturen (BK Klon 3a) rücktitrierbar. Dies weist auf prinzipielle biologische Unterschiede der beiden Stämme im Infektions- und Replikationsverhalten hin. Gleichzeitig zeigt dieses Ergebnis, dass NZ2 in einem breiteren Spektrum von Zellen als D1701 reproduziert werden kann, woraus sich die Möglichkeit eines auf humanen Zellen beruhenden Produktionsverfahrens ergibt.
4. b) Passagierbarkeit von NZ2 und D1701 auf WI-38 Zellen und BK Klon 3a Zellen
   Wesentliche biologische Unterschiede zwischen NZ2 und D1701 Stämmen werden auch durch die in Figur 8 und Figur 9 dargestellten Experimente deutlich. Figur 8 zeigt den Virustiter in Passagierungsversuchen von D1701 auf bovinen BK Klon 3a Zellen und auf humanen WI-38 Zellen über die Anzahl der Passagen. Es ist erkennbar, dass D1701 auf BK Klon 3a Zellen fortgesetzt passagierbar ist, auf humanen WI-38 Zellen jedoch nicht.
   Anders verhält es sich mit dem NZ2 Stamm. Dieser Stamm ist über mehrere Tage sowohl auf BK Klon 3a Zellen als auch auf WI-38 Zellen fortgesetzt passagierbar (Figur 9).
   Auch diese Ergebnisse weisen auf deutliche Unterschiede im Infektions- und Replikationsverhalten von NZ2 und D1701 hin.
4. c) Dosisabhängige Wirkung von NZ2 im Herpesvirus-Challenge-Test nach Passagierung auf WI-38
   Zur Untersuchung der immunstimulatorischen Eigenschaften von NZ2 Stämmen, welche auf WI-38 Zellen passagiert worden sind, sind Herpesvirus-Challenge-Tests an Mäusen durchgeführt worden. Es wurden drei Gruppen von jeweils 10 Versuchstieren mit jeweils 1x10⁴ TCID₅₀, 5x10⁴ TCID₅₀ und 1x10⁵ TCID₅₀ behandelt. eine Kontrollgruppe erhielt ein Placebo. Die Überlebensrate der vier Versuchsgruppen ist in Figur 10 über die Zeit nach der Herpesvirusinfektion dargestellt. Überraschenderweise wurde festgestellt, dass NZ2 durch die Passage auf WI-38 Zellen seine immunstimulatorischen Eigenschaften nicht verloren hat.
   Alle experimentellen Ergebnisse dieses Beispiels weisen auf grundlegende biologische Unterschiede im Infektions- und Replikationsverhalten von NZ2 und D1701 hin. Es wurde überraschenderweise gefunden, dass die Herstellung von NZ2 zur Verwendung als Immunmodulator im Gegensatz zu D1701 nicht an Rindernierenzellen als Produktionszelllinie gebunden ist.

Unter Zugrundelegung der bekannten Zusammenhänge vom Einfluss einer Thl Immunantwort auf latente und chronisch persistente Virusinfektionen^{4,5)} sowie proliferative Erkrankungen, z.B. Krebs^{8,9)} und der im Vergleich zu Parapoxvirus ovis, Strain D 1701 besseren immunmodulatorischen Eigenschaften des Parapoxvirus ovis, Strain NZ 2 ist der Einsatz von Immunmodulatoren auf der Basis von Parapoxvirus ovis, Strain NZ 2 oder einer der o.g. Stämme als Monotherapie oder in Kombination mit biologisch aktiven, z.B. antiviralen, niedermolekularen Verbindungen an Mensch und Tier möglich und von therapeutischem Nutzen zur antiviralen Therapie von Infektionen mit dem Hepatitis B Virus; dem Hepatitis C Virus oder allen anderen Erregem aus der Gruppe der Hepatitis verursachenden Viren sowie anderen viralen Infektionen der inneren Organe, sowie Infektionen, auch in Begleitung anderer Erkrankungen, mit den verschiedenen Typen des Herpes simplex Virus (HSV), den verschiedenen Typen von humanem Papillomvirus (HPV), dem humanem Immundefizienzvirus (HIV), dem Varizella Zoster Virus, dem humanem Cytomegalievirus (HCMV), sowie den entsprechenden Viruserkrankungen beim Tier.

Des weiteren können mit den oben genannten Stämmen von Parapoxvirus ovis aufgrund des gezeigten Wirkmechanismus insbesondere die folgenden prophylaktischen oder therapeutischen Behandlungen erfolgversprechend durchgeführt werden:

Verhinderung von Rekurrenzen bei Herpesvirus-Infektionen, Metaphylaxe, d.h. Verhinderung der Etablierung von viralen Infektionen (z.B. HIV), wenn unmittelbar nach der Exposition mit dem Mittel behandelt wird ⁷⁾. Die Behandlung von Krebs ist aufgrund des Wirkmechanismus ebenfalls möglich ^{8,9)}.

Je nach klinischer Fragestellung wird das Therapeutikum auf der Basis von Parapoxvirus systemisch, also beispielsweise intramuskulär, subcutan, intraperitoneal, intravenös, per os, oder lokal appliziert. Das Parapoxvirus liegt dabei entweder aufgereinigt und lyophilisiert vor und/oder wird unmittelbar vor der Applikation in einem geeigneten Lösungsmittel suspendiert oder es liegt in einer anderen geeigneten Formulierung vor oder es liegt in einer magensaftresistenten oder einer anderen oralen Applikationsform vor.

Geeignete Zubereitungen können ebenfalls aus durch Passagierung und/oder Adaptation an bestimmte Zellen, beispielsweise WI-38, MRC-5 oder Vero-Zellen, erhaltenen Abkömmlingen von NZ2 und den anderen genannten Stämmen bzw. Teilen oder Bruchstücken von NZ2 und den anderen genannten Stämmen oder diesen Abkömmlingen hergestellt werden. Unter Teilen sind mittels geeigneter Vektoren, beispielsweise Vaccinia, in geeigneten Systemen, beispielsweise Fibroblastenzellkulturen, exprimierte genomische oder subgenomische Fragmente zu verstehen. Unter Bruchstücken versteht man die durch biochemische Aufreinigung, beispielsweise Chromatografie, erhaltenen Fraktionen physikalisch, beispielsweise durch Einwirkung von Ultraschall, aufgeschlossener Partikel.

Mehrere Applikationen oder Langzeitbehandlung nach Zeitschemen, die den Erfordernissen der klinischen Fragestellung entsprechen, können dabei notwendig sein

So erweist sich bei der Krebstherapie beispielsweise (jedoch ohne Einschränkung) die Anwendung nach folgendem Schema als besonders erfolgversprechend:

Intramuskuläre Applikation von jeweils 10⁶ bis 10⁷ TCID₅₀ (Tissue Culture Infective Dosage) für 4 Wochen an jedem 3. Tag gefolgt von 2 Wochen Pause; erneute intramuskuläre Applikation von jeweils 10⁶ bis 10⁷ TCID₅₀ für 4 Wochen an jedem 3. Tag gefolgt von 2 Wochen Pause; emeute intramuskuläre Applikation von jeweils 10⁶ bis 10⁷ TCID₅₀ für 4 Wochen an jedem 3. Tag gefolgt von 2 Wochen Pause; je nach Schweregrad und Heilungserfolg können diese Cyclen durch weitere Cyclen ergänzt werden; alternativ kann auch ein Schema unter dem die Formulierung jeden 4. bis 5. Tag über mindestens 3 Monate appliziert wird, indiziert sein.

Bei chronischer Virusinfektion werden beispielsweise 10⁶ bis 10⁷ TCID₅₀ der Formulierung subcutan im Bauchbereich oder intramuskulär im Bereich des Deltoid oder Quadrizeps jeden 3. Tag, insgesamt 5 mal, appliziert. Abweichungen von diesem Schema können entsprechend den Bedürfnissen, die sich aus der Erkrankung ergeben, vorgenommen werden. Zur Prophylaxe von Erkältungen ist mit der Formulierung zu gurgeln und diese Anwendung täglich zu wiederholen, solange wie das Ansteckungsrisiko besteht.

Für die Vorbeugung von Infektionen nach oralchirurgischen Eingriffen (z.B. Zahnoperation) ist am Vorabend vor dem Eingriff 1-2 min. zu gurgeln.

### Literatur:

- 1.: Guidotti, L.G., Borrow, P., Hobbs, M.V., Matzke, B., Gresser, I., Oldstone, M.B.A., and Chisari, F.V. (1996): Viral cross talk: Intracellular inactivation of the hepatitis B virus during an unrelated viral infection of the liver. Proc. Natl. Acad. Sci. USA. 93:4589-4594.
- 2.: Guidotti, L.G., Ando, K., Hobbs, M.V., Ishikawa, T., Runkel, L., Schreiber, R.D., and Chisari, F.V. (1994): Cytotoxic T lymphocytes inhibit hepatitis B virus gene expression by a noncytolytic mechanism in transgenic mice. Proc. Natl. Acad. Sci. USA. 91:3764-3768.
- 3.: Steinmassl,G., G.Wolf (1990): Bildung von Interleukin 2 und Interferon- durch mononukleäre Leukozyten des Schweines nach in vitro-Stimulation mit verschiedenen Viruspräparaten. J.Vet.Med.B37,5,321-331.
- 4.: P. Lucin, S. Jonjic, M. Messerle, B. Polic, H. Hengel, U.H. Koszinowski (1994): Late-Phase inhibition of murine cytomegalovirus replication by synergistic action of interferon gamma and tumor necrosis factor alpha. J. Gen. Virol 75:101-110; P.M.
- 5.: Smith, R.M. Wolcott, R. Chervenak, S.R. Jennings (1994): Control of acute cutaneous herpes-simplex virus-Infection - T-cell mediated viral clearance is dependent upon interferon gamma. Virology 202 (1):76-88].
- 6.: Y. Kawanashi, N. Hayashi, K. Katayama, K. ueda, T. Takehara, E. Miyoshi, E. Mita, A. Kasahara, H. Fusamoto, T. Kamada (1995): Tumor necrosis factor alpha and interferon gamma inhibit synergistically viral replication in hepatitis B virus replicating cells. J. Medical Virology 47 (3):272-277.
- 7.: Dhawan, S., L.M. Wahl, A. Heredia, Y.H. Zhang, J.S. Epstein, M.S. Meltzer, I.K. Hewlett (1995): Interferon gamma inhibits HIV-induced invasiveness of Monocytes. J. Leukocyte Biology, 58 (6):713-716.
- 8.: J.F. Bromberg, C.M. Horvath, Z.L. Wen, R.D. Schreiber, J.E.Darnell (1996): Transcriptionally active statl is required for the antiproliferative effects of both interferon alpha and interferon gamma. PNAS 93(15):7673-7678.
- 9.: M.Klouche, H.Kirchner, F.Holzel (1994): Antiproliferative effects of interferon gamma in combination with alpha-difluoromethylornithine on human carcinoma cell cultures. J.Cancer Research and Clinical Oncology 120(12):706].

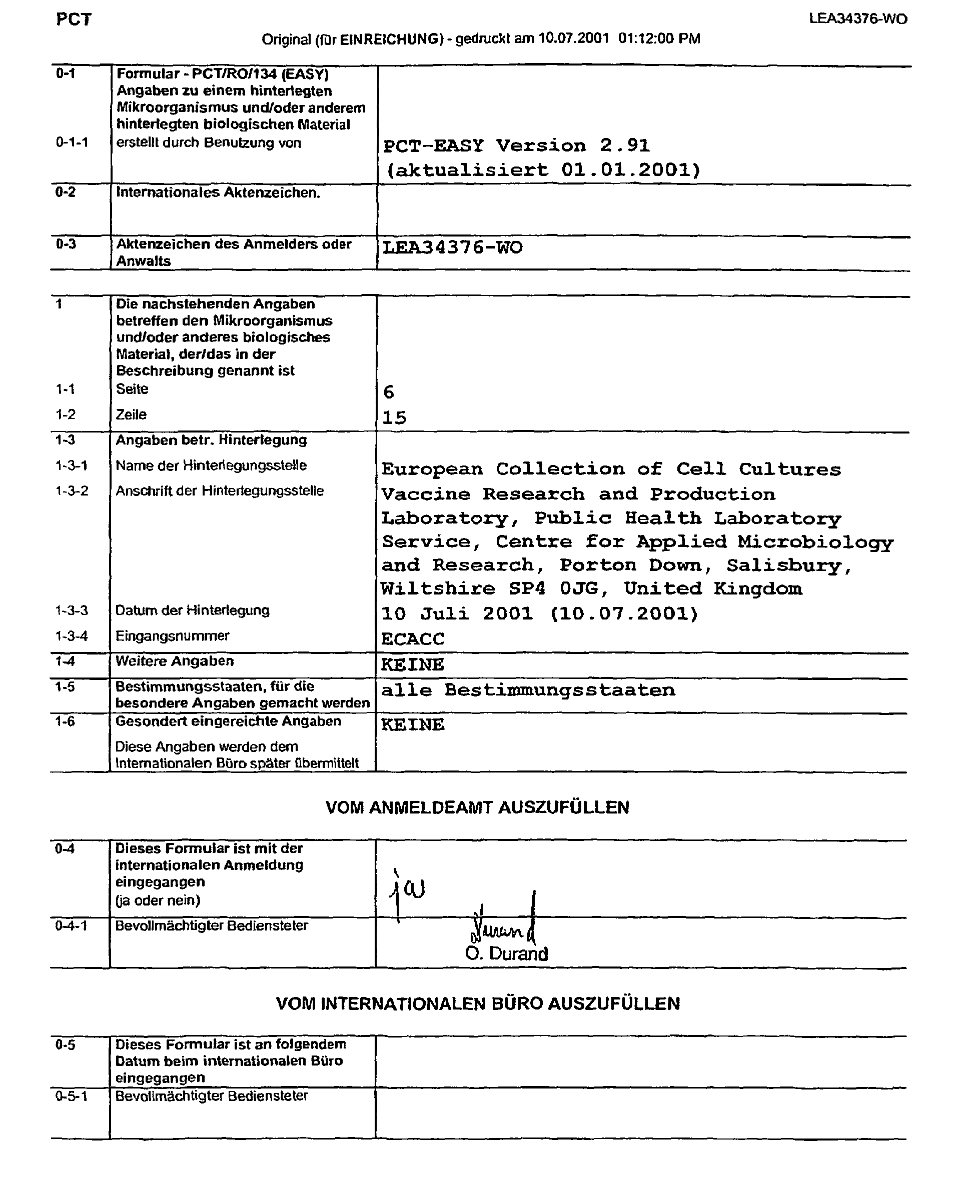

## Patentansprüche

1. Verwendung von Viren, die taxonomisch zum Stamm NZ2, von Parapoxvirus ovis gehören zur Herstellung von Arzneimitteln gegen virale Infektionen und Krebs bei Mensch und Tier.

2. Verwendung von durch Passagienmg oder Adaptation an geeigneten Zellsystemen wie beispielsweise humanen Zellen wie WI-38, MRC-5, Vero-Zellen, bovinen Zellen wie BK-K13A47/Reg oder MDBK, und ovinen Zellen wie MDOK, erhältlichen Abkömmlingen der Viren gemäss Anspruch 1 zur Herstellung von Arzneimitteln gegen virale Infektionen und Krebs bei Mensch und Tier.

3. Verwendung von Teilen oder Bruchstücken der Viren gemäss Anspruch 1 oder 2, wobei unter Teilen mittels geeigneter Vektoren wie Vacciniaviren in geeigneten Systemen wie z.B. Fibroblastenzellkulturen exprimierte genomische oder subgenomische Fragmente und unter Bruchstücken die durch biochemische Aufreinigung wie Chromatografie erhältlichen Fraktionen der exprimierten oder physikalisch aufgeschlossenen viralen Partikel zu verstehen sind und wobei die Teile oder Bruchstücken der Viren eine paraspezifische Immunantwort induzieren, zur Herstellung von Arzneimitteln gegen virale Infektionen und von Arzneimitteln gegen virale Infektionen und Krebs bei Mensch und Tier.

4. Verwendung entsprechend einem der Anspruche 1-3 zur Herstellung von Arzneimitteln und pharmazeutischen Zubereitungen als Immuntherapeutika oder bei akuten und chronischen viralen Infektionen des Respirationstraktes und der inneren Organe.

5. Verwendung entsprechend einem der Ansprüche 1-3 zur Herstellung von Arzneimitteln und pharmazeutischen Zubereitungen zur Verhinderung oder Vorbeugung von Infektionskrankheiten nach Stress sowie bei der Infektionsprophylaxe im Rahmen von Operationen und zahnärztlichen Eingriffen.

6. Verwendung entsprechend einem der Ansprüche 1-3 zur Herstellung von Arzneimitteln und pharmazeutischen Zubereitungen für die Verwendung bei der Infektionsmetaphylaxe oder Therapie akuter viraler Infektionen des Respirationstraktes, der Papillomvirusinfektionen, der Infektion mit Herpesviren, der HIV-Infektion, der Virusinfektion innerer Organe wie beispielsweise der Infektion mit Hepatitisviren.

7. Verwendung entsprechend einem des Ansprüche 1-3 zur Herstellung von Arzneimitteln und pharmazeutischen Zubereitungen für die Verwendung gegen proliferative Erkrankungen der inneren Organe, der Haut, des Blutes, des Zentralnervensystems und seiner Anhangsgebilde einschließlich des Auges, einschließlich Krebs.

8. Verwendung entsprechend einem der Ansprüche 1-3 in Kombination mit anderen Mitteln zur Herstellung von Arzneimitteln und pharmazeutischen Zubereitungen zur antiviralen- oder Krebstherapie bei Mensch und Tier.

9. Verwendung entsprechend einem der Ansprüche 1-3 in Kombination mit anderen Mitteln zur Herstellung von Arzneimitteln und pharmazeutischen Zubereitungen für die orale Applikation und/oder in einer magensaftresistenten Formulierung für die orale Applikation.

## Claims

1. Use of viruses which belong taxonomically to the Parapoxvirus ovis strain NZ2, for producing medicaments directed against viral infections and cancer in humans and animals.

2. Use of descendants of the viruses according to Claim 1, which descendants are obtainable by passaging or adaptation to suitable cell systems, for example human cells, such as WI-38 and MRC-5, Vero cells, bovine cells, such as BK-K13A47/Reg or MDBK, and ovine cells, such as MDOK, for producing medicaments which are directed against viral infections and cancer in humans and animals.

3. Use of parts or fragments of the viruses according to Claim 1 or 2, where parts are to be understood as being genomic or subgenomic fragments which are expressed with the aid of suitable vectors, such as vaccinia viruses, in suitable systems, such as fibroblast cell cultures, and fragments are to be understood as being the fractions, which are obtained by biochemical purification, such as chromatography, of the expressed or physically disrupted viral particles, and where the parts of fragments of the viruses induce a paraspecific immunoresponse, for producing medicaments which are directed against viral infections and cancer in humans and animals.

4. Use of one of the strains of Parapoxvirus ovis according to Claims 1 - 3 for producing medicaments and pharmaceutical preparations as immunotherapeutic agents or immunoprophylactic agents for autoimmune diseases and for acute and chronic viral infections of the respiratory tract and the internal organs.

5. Use according to one of Claims 1 - 3 for producing medicaments and pharmaceutical preparations for preventing or averting infectious diseases following stress and also in connection with infection prophylaxis within the context of operations and dental interventions.

6. Use according to one of Claims 1 - 3 for producing medicaments and pharmaceutical preparations for use in infection metaphylaxis or the therapy of acute viral infections of the respiratory tract, of papilloma virus infections, of infection with herpesviruses, of HIV infection, or of viral infection of internal organs, such as infection with hepatitis viruses.

7. Use according to one of Claims 1 - 3 for producing medicaments and pharmaceutical preparations for use against proliferative diseases of the internal organs, of the skin, of the blood, of the central nervous system and its appended structures including the eye, and including cancer.

8. Use according to one of Claims 1 - 3 in combination with other remedies for producing medicaments and pharmaceutical preparations for antiviral or cancer therapy in humans and animals.

9. Use according to one of Claims 1 - 3 in combination with other remedies for producing medicaments and pharmaceutical preparations for oral administration and/or in a gastric juice-resistant formulation for oral administration.

## Revendications

1. Utilisation de virus qui appartiennent du point de vue taxonomique à la souche NZ2 de Parapoxvirus ovis pour la fabrication de médicaments dirigés contre des infections virales et contre le cancer en médecine humaine et en médecine vétérinaire.

2. Utilisation de descendants des virus obtenus par passage ou adaptation sur des systèmes cellulaires convenables tels que, par exemple, des cellules humaines comme WI-38, MRC-5, cellules véro, cellules bovines telles que BK-K13A47/Reg ou MDBK, et cellules ovines telles que MDOK, suivant la revendication 1, pour la fabrication de médicaments dirigés contre des infections virales et contre le cancer en médecine humaine et en médecine vétérinaire.

3. Utilisation de parties ou segments des virus suivant la revendication 1 ou 2, en considérant comme parties des fragments génomiques ou subgénomiques exprimés au moyen de vecteurs convenables tels que des virus de vaccins dans des systèmes convenables tels que, par exemple, des cultures cellulaires de fibroblastes et, comme segments, les fractions obtenues par une purification biochimique telle qu'une chromatographie, des particules virales exprimées ou désagrégées physiquement, et les parties ou segments des virus induisant une réponse immunitaire paraspécifique, pour la fabrication de médicaments dirigés contre des infections virales et contre le cancer en médecine humaine et en médecine vétérinaire.

4. Utilisation suivant l'une des revendications 1 à 3 pour la fabrication de médicaments et de préparations pharmaceutiques comme moyens immunothérapiques, ou en cas d'infections virales aiguës et chroniques des voies respiratoires et des organes internes.

5. Utilisation suivant l'une des revendications 1 à 3 pour la fabrication de médicaments et de préparations pharmaceutiques pour inhiber ou prévenir des maladies infectieuses après un stress ainsi que dans la prophylaxie contre les infections dans le cadre d'opérations et d'interventions odontologiques.

6. Utilisation suivant l'une des revendications 1 à 3, pour la fabrication de médicaments et de préparations pharmaceutiques destinés à être utilisés dans la métaphylaxie anti-infectieuse ou la thérapie d'infections virales aiguës des voies respiratoires, d'infections à virus du papillome, d'infection à virus de l'herpès, d'infection à virus VIH, d'infection virale d'organes internes, par exemple de l'infection à virus de l'hépatite.

7. Utilisation suivant l'une des revendications 1 à 3, pour la fabrication de médicaments et de préparations pharmaceutiques destinés à être utilisés contre des maladies prolifératives des organes internes, de la peau, du sang, du système nerveux central et de ses appendices, y compris l'oeil, ainsi que contre le cancer.

8. Utilisation suivant l'une des revendications 1 à 3 en combinaison avec d'autres moyens pour la fabrication de médicaments et de préparations pharmaceutiques destinés à la thérapie antivirale ou anticancéreuse en médecine humaine et en médecine vétérinaire.

9. Utilisation suivant l'une des revendications 1 à 3 en combinaison avec d'autres moyens pour la fabrication de médicaments et de préparations pharmaceutiques destinés à l'administration orale et/ou dans une formulation résistant aux sucs gastriques pour l'administration orale.


### SEQUENZPROTOKOLL

<110> Bayer AG
<120> Verwendung von Stämmen des Parapoxvirus ovis zur Herstellung ......von antiviralen Arzneimitteln
<130> Parapoxvirus ovis Strain NZ2
<140>
<141>
<160> 11
<170> PatentIn Ver. 2.1
<210> 1
<211> 25
<212> DNA
<213> Mus sp.
<220>
<223> beta-Actin primer, antisense
<400> 1
   taaaacgcag ctcagtaaca gtccg 25
<210> 2
<211> 25
<212> DNA
<213> Mus sp.
<220>
<223> beta-Actin primer, sense
<400> 2
   tggaatcctg tggcatccat gaaac 25
<210> 3
<211> 26
<212> DNA
<213> Hepatitis B virus
<220>
<223> HBV sonde, ayw-613t
<400> 3
   ccatcatcct gggctttcgg aaaatt 26
<210> 4
<211> 19
<212> DNA
<213> Hepatitis B virus
<220>
<223> HBV primer, ayw-670r (antisense)
<400> 4
   aggagaaacg ggctgaggc 19
<210> 5
<211> 21
<212> DNA
<213> Hepatitis B virus
<220>
<223> HBV primer, ayw-570f (sense)
<400> 5
   ctgtaccaaa ccttcggacg g 21
<210> 6
<211> 24
<212> DNA
<213> Mus sp.
<220>
<223> Primer IFN-gamma (antisense)
<400> 6
   gtcacagttt tcagctgtat aggg 24
<210> 7
<211> 26
<212> DNA
<213> Mus sp.
<220>
<223> Primer IFN-gamma (sense)
<400> 7
   agcggctgac tgaactcaga ttgtag 26
<210> 8
<211> 30
<212> DNA
<213> Mus sp.
<220>
<223> Primer IL-15 (antisense)
<400> 8
   cgtgttgatg aacatttgga caatgcgtat 30
<210> 9
<211> 29
<212> DNA
<213> Mus sp.
<220>
<223> Primer IL-15 (sense)
<400> 9
   gccaactgga tagatgtaag atatgacct 29
<210> 10
<211> 25
<212> DNA
<213> Mus sp.
<220>
<223> Primer TNF-alpha (antisense)
<400> 10
   acattcgagg ctccagtgaa ttcgg 25
<210> 11
<211> 25
<212> DNA
<213> Mus sp.
<220>
<223> Primer TNF-alpha (sense)
<400> 11
   ggcaggtcta ctttggagtc attgc 25